Europäisches Patentamt

European Patent Office

Office européen des brevets

(19)

(11) Numéro de publication : **0 323 333 B1**

# (12) FASCICULE DE BREVET EUROPEEN

(45) Date de publication du fascicule du brevet :
06.11.91 Bulletin 91/45

(51) Int. Cl.⁵ : **A61M 25/00, A61F 2/02**

(21) Numéro de dépôt : **88403304.4**

(22) Date de dépôt : **23.12.88**

(54) **Filtre anti-embolique.**

(30) Priorité : 31.12.87 FR 8718472
15.06.88 FR 8808031

(43) Date de publication de la demande :
05.07.89 Bulletin 89/27

(45) Mention de la délivrance du brevet :
06.11.91 Bulletin 91/45

(84) Etats contractants désignés :
AT BE CH DE ES FR GB GR IT LI LU NL SE

(56) Documents cités :
EP-A- 0 121 447
DE-A- 3 347 150
FR-A- 2 606 642
GB-A- 2 020 557
US-A- 4 354 491

(73) Titulaire : BIOMAT(S.a.r.l.)
17, rue Maryse Bastie
F-91430 Igny (FR)

(72) Inventeur : Lahille, Michel André
17, rue de Favreuse
F-91430 Vauhallan (FR)
Inventeur : Dibie, Alain Jean-Marie
37, avenue de Lôwendal
F-75015 Paris (FR)

(74) Mandataire : Martinet & Lapoux
BP 405
F-78055 St. Quentin en Yvelines Cédex (FR)

EP 0 323 333 B1

Jouve, 18, rue Saint-Denis, 75001 PARIS

## Description

La présente invention concerne un filtre anti-embolique pour la prévention des embolies sanguines, et plus particulièrement un filtre veineux-cave contre les embolies cruoriques.

Le filtre a notamment pour mission de stopper la migration de thrombus venant de la circulation veineuse périphérique des membres inférieurs ou du petit bassin et se dirigeant vers les artères pulmonaires, lieu où les thrombus pourraient provoquer par obstruction des grosses branches, des embolies sévères souvent mortelles.

Divers filtres pour veine cave inférieure ont été développés et sont décrits dans l'article de J. DRILLER et al., intitulé "New percutaneous caval filter device for pulmonary thromboembolism", dans Medical and Biological Engineering, November 1976, New York, pages 629 à 635.

Un tel filtre a généralement un corps expansible qui est pliable en une forme générale cylindrique pour l'introduire dans un cathéter et le pousser dans celui-ci jusqu'au lieu d'implantation du filtre, dans la veine cave inférieure. Le corps du filtre sortant du cathéter se déploie ensuite en une forme de révolution dans la veine afin que le filtre agrandisse la section de la veine et constitue un obstacle à tout corps étranger dangereux, tel que caillot sanguin, susceptible de provoquer une embolie pulmonaire.

L'ombrelle trouée de MOBIN-UDDIN décrite dans le US-A-3540431 est un filtre endoveineux qui nécessite l'abord chirurgical de la veine jugulaire interne sous anesthésie locale. Le filtre de MOBIN-UDDIN offre plusieurs inconvénients. Pour insérer le filtre en ombrelle, celui-ci doit être replié et vissé dans une capsule tubulaire d'un adaptateur qui est poussé au moyen d'un cathéter tubulaire flexible jusqu'à l'endroit d'implantation. L'encombrement de l'adaptateur est important et ne permet pas l'introduction du filtre à travers des veines jugulaires étroites et tortueuses. En outre, le filtre en ombrelle peut migrer à distance ou basculer. Le filtre entraîne, de façon plus ou moins différée, une thrombose fréquente, provoquant un syndrome de stase veineuse inférieure.

Le filtre de Lazar J. GREENFIELD, plus récent, est composé d'un ensemble de fils ondulés en acier inoxydable qui sont reliés entre eux par une capsule métallique et qui sont déployés suivant des génératrices d'un cône, comme des tiges d'un parapluie. Il offre des inconvénients analogues à ceux du filtre de MOBIN-UDDIN. Toutefois, le filtre de GREENFIELD peut être introduit par la veine fémorale ou la veine jugulaire. Ce filtre nécessite une dénudation chirurgicale ou un abord percutané à l'aide d'un introducteur de gros calibre de type 24F ayant un diamètre de 7,92 mm. La pluralité de crochets d'ancrage radiaux du filtre due aux extrémités libres acérées et recourbées des fils apparaît être traumatique pour la paroi veineuse. Les malpositions du filtre dans la veine cave et les migrations du filtre vers le coeur ne sont pas exceptionnelles.

Un troisième filtre endoveineux, dit filtre de GUNTHER, est constitué par une première enveloppe (corbeille) flexible tubulaire composée de fils métalliques prédéformés torsadés axialement, et d'un ensemble de fils courbés fixé à une extrémité de l'enveloppe et analogue un filtre de GREENFIELD. Lorsque le filtre est déployé, l'enveloppe a une section longitudinale délimitée sensiblement par deux alternances sinusoïdales opposées. L'enveloppe compressée radialement du filtre de GUNTHER offre encore un diamètre relativement grand qui nécessite l'emploi d'un cathéter-introducteur de grande taille, du type 10F correspondant à un diamètre de l'ordre de 3,3 mm.

Tous ces filtres connus exigent en outre un matériel complexe qui est spécialement conçu pour introduire le filtre dans un cathéter particulier de fort calibre 9F à 24F. En effet, le filtre doit être tiré au moyen d'un crochet dans un fourreau, dit adaptateur ou cartouche, afin de réduire sa section à la section interne du cathéter, puis le fourreau est enfiché dans une extrémité proximale du cathéter afin de pousser le filtre dans le cathéter. Quel que soit le filtre connu, sa section réduite dans le cathéter dépend du grand nombre de fils métalliques serrés les uns contre les autres, ce qui limite l'utilisation de ces filtres à des veines de relativement grand diamètre, ou tout au moins, accroît la durée et les difficultés de l'intervention chirurgicale.

Le document EP-A-0121447 décrit un filtre contre les embolies, constitué d'un filtre prédéformé avec des ondulations principales et secondaires dans des plans différents.

La présente invention vise à remédier aux principaux inconvénients des filtres anti-emboliques précités. En particulier, l'invention vise à fournir un filtre anti-embolique qui offre une très faible section pour son introduction sans risque d'accrochage dans un cathéter de petite taille permettant l'utilisation des veines des membres supérieurs. Le filtre requiert un matériel simple et bon marché pour l'introduire dans un cathéter standard, et qui, lorsqu'il est implanté, est par nature équilibré et stable dans la veine, sans risque de migration.

A cette fin, un filtre anti-embolique selon l'invention, à implanter dans un canal physiologique, est tel que défini dans la revendication 1.

Le fil peut être étalé à un état pratiquement rectiligne afin d'être introduit aisément dans un cathéter de faible diamètre externe typiquement égal à 2,3 mm, par simples poussées manuelles successives. Tout abord veineux peut être choisi, tel que veine jugulaire, veine fémorale, veine céphalique ou veine humérale. Le faible diamètre du cathéter et sa souplesse qui n'est pas amoindrie par l'enfilage du filtre filiforme dans le cathéter, permet de franchir, sans agresser la paroi veineuse, diverses sinuosités des veines. Par exemple, le cathéter enfilé dans la veine humérale d'un membre supérieur peut franchir l'oreillette droite pour positionner exactement l'extrémité proximale du cathéter à l'endroit désiré, dans la veine cave inférieure.

Grâce au pouvoir élastique du fil, le fil retrouve sa configuration primitive en deux boucles croisées de préférence elliptiques ou oblongues dès que le fil sort du cathéter dans le canal physiologique, tel que la veine cave. Les boucles sont développées suivant la direction longitudinale de la veine. Le caractère plat du filtre ainsi détendu et de diamètre supérieur à celui de la veine impose une section plate transversale de la veine, sensiblement proche d'une ellipse ayant une excentricité proche de l'unité, qui est entrecoupée par les divers croisements des arcs des deux boucles superposées ce qui stoppe toute migration possible de caillots sanguins jugés de taille dangereuse.

Les boucles sont déployées suivant la direction du flux sanguin longitudinal à la veine. Toutefois, grâce à la quasi-symétrie du filtre, celui-ci est stable. En effet, les boucles peuvent être inscrites dans un carré. Chacune des boucles est tangentielle à deux côtés parallèles du carré, c'est-à-dire en pratique, les boucles s'appuient en quatre points de contact de la paroi veineuse, symétriques deux-à-deux par rapport au centre du filtre. Ces points de contact ne blessent pas la paroi veineuse et seront intimement liés à celle-ci par endothélialisation quelques jours après l'implantation du filtre.

Conjointement à cette symétrie du filtre s'ajoute le fait que le fil est prédéformé initialement en deux boucles, d'une première extrémité libre de la première boucle vers une seconde extrémité libre de la seconde boucle, suivant un même sens de rotation autour du centre du filtre, sans contrarier le sens du tréfilage du fil. Par suite, la compression exercée par la paroi veineuse sur le filtre ne tend pas à étaler le filtre afin que celui-ci migre dans la veine, comme pour certains filtres selon la technique antérieure.

Selon d'autres caractéristiques de l'invention, les extrémités du filtre comprennent chacune une masselotte de révolution, cylindrique ou tronconique. Les masselottes sont situées au voisinage du centre du filtre, latéralement à celui-ci. En outre, la seconde extrémité du filtre, dite extrémité distale, introduite en dernier dans le cathéter et la veine a de préférence une direction sensiblement rectiligne et inclinée à 45° par rapport au plan des boucles du filtre. La seconde extrémité comporte, à la suite de la masselotte, une pointe acérée, qui pénètre sensiblement en biais dans la paroi veineuse sans toutefois la traverser, grâce au rôle de butée de la masselotte. La pointe acérée contribue à augmenter la stabilité du filtre.

L'invention a trait également à un poussoir destiné particulièrement à recevoir la seconde extrémité du filtre afin de pousser le filtre dans le cathéter et de l'expulser dans la veine, mais également, si besoin, de le tirer vers l'arrière dans le cathéter pour vérifier et ajuster la position du filtre. Le poussoir est tel que défini dans les revendications 18 à 20. Par suite, la première extrémité du filtre qui ne comprend qu'une masselotte, guide le glissement doux du fil dans le cathéter, et la pointe acérée à la seconde extrémité n'entrave pas la poussée du poussoir dans le cathéter, du fait que la seconde extrémité est totalement encastrée dans le poussoir.

D'autres avantages et caractéristiques de l'invention apparaîtront plus clairement à la lecture de la description suivante de plusieurs réalisations préférées de l'invention en référence aux dessins annexés correspondants dans lesquels :

— la Fig. 1 est une vue longitudinale à plat d'un filtre anti-embolique à deux boucles elliptiques orthogonales selon l'invention, placé dans une section longitudinale d'une veine ;

— la Fig. 2 est une vue longitudinale à plat d'un filtre anti-embolique à deux boucles oblongues orthogonales selon l'invention, placé dans une section longitudinale d'une veine ;

— la Fig. 3 est une vue transversale du filtre et de la veine de la Fig. 2 ;

— la Fig. 4 est une vue longitudinale détaillée d'une première extrémité de première boucle d'un filtre selon l'invention ;

— la Fig. 5 est une vue longitudinale détaillée d'une seconde extrémité de seconde boucle d'un filtre selon l'invention ;

— les Figs. 6 et 7 sont respectivement des vues longitudinales de côté et de dessus d'un poussoir selon l'invention, comportant une seconde extrémité de filtre et inclus dans un cathéter ; et

— la Fig. 8 est une vue schématique en perspective du poussoir sortant partiellement d'une extrémité proximale du cathéter, lors du largage d'un filtre selon l'invention dans une veine.

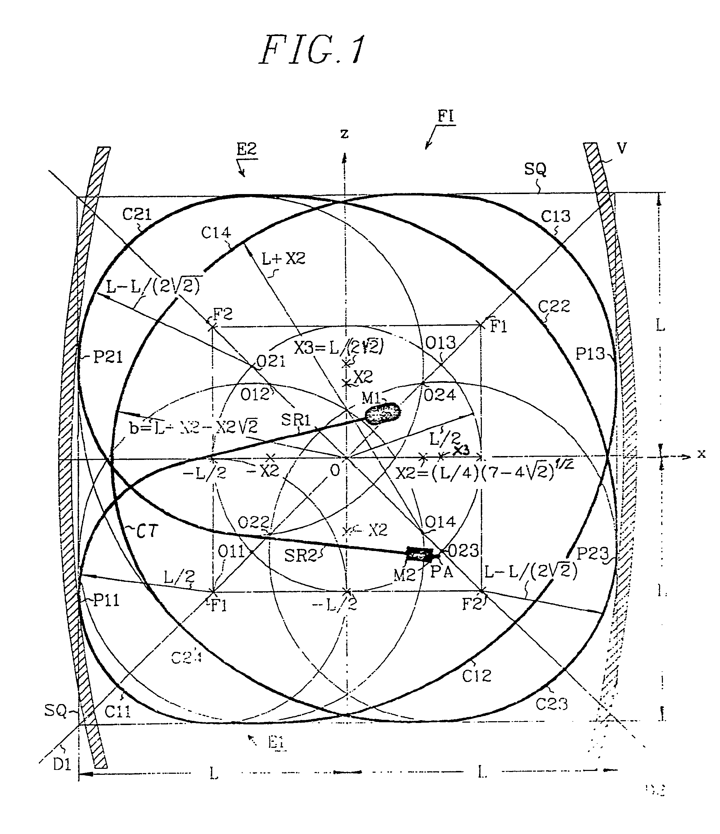

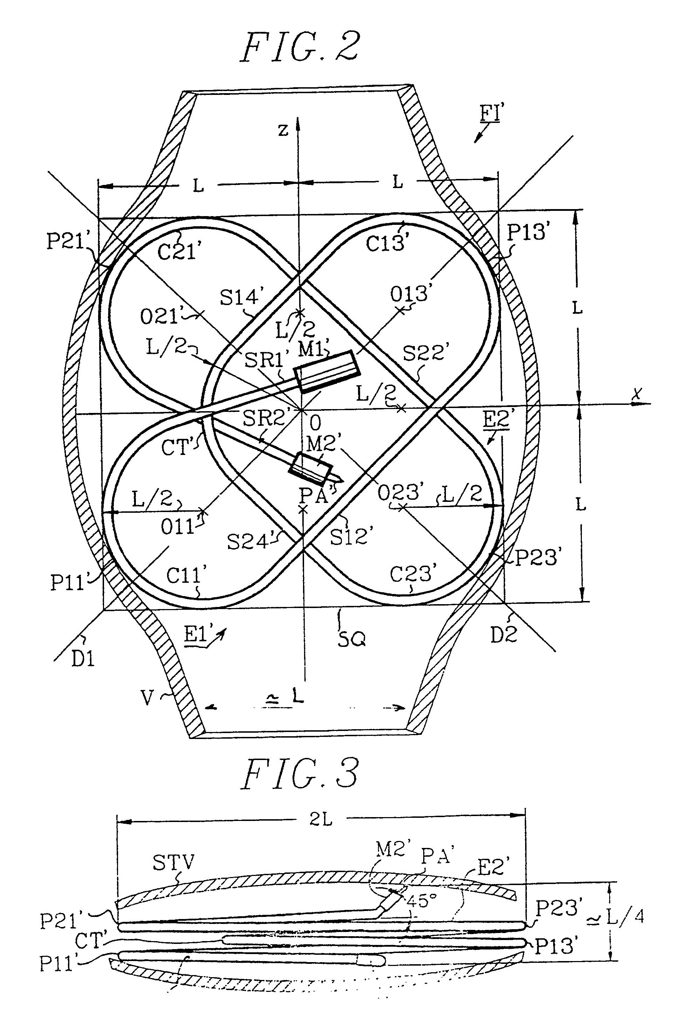

Selon les deux réalisations illustrées aux Figs. 1 et 2, un filtre anti-embolique selon l'invention est réalisé à partir d'un fil métallique radio-opaque, flexible et élastique, de préférence en alliage cobalt-chrome, qui a été soumis à une prédéformation primitive. Les propriétés de mémorisation de la prédéformation par le fil permettent de déployer le fil, par exemple à un état rectiligne en l'étirant par les deux extrémités de celui-ci, et, après relâchement des extrémités du fil, de retrouver la configuration de la prédéformation grâce à l'effet de ressort du fil.

Selon la première réalisation montrée à la Fig. 1, la prédéformation primitive confère au fil une configuration en deux boucles plates ovales, très voisines de deux ellipses sensiblement identiques E1 et E2 ayant des grands axes D1 et D2 orthogonaux. Le filtre FI ainsi formé est inscrit dans un carré SQ de côté 2L dont les diagonales sont confondues avec les grands axes D1 et D2, et le centre O est confondu avec ceux des deux boucles elliptiques E1 et E2. Dans la Fig. 1, la première boucle E1 est superposée sur la seconde boucle E2.

Afin de fixer les idées, la géométrie du filtre FI est indiquée en détail ci-après en référence à un repère cartésien centré en O et ayant un axe d'abscisse Ox et un axe de cote Oz qui sont respectivement horizontal et vertical et qui sont bissecteurs des diagonales D1 et D2 du carré SQ.

La première boucle E1 peut être décomposée à partir d'une première extrémité libre M1 du filtre FI située au voisinage du centre O dans le premier quadrant du repère Ox, Oz, en :

— un segment rectiligne ou sensiblement convexe SR1 tangent à un cercle C11 ayant un centre O11 de coordonnées $-L/2$, $-L/2$ et un rayon égal à $L/2$,

— un arc du cercle C11 ayant une longueur sensiblement inférieure à la demi-circonférence du cercle C11 et se terminant sensiblement au point de tangente du cercle C11, sur le côté inférieur du carré SQ,

— un arc de cercle C12 traversant sensiblement en diagonale le quatrième quadrant du repère Ox, Oz, ayant un centre O12 de coordonnées $-X2 = -(L/4)(7-\sqrt{2})^{1/2}$, X2, et un rayon égal à $L + X2$, l'arc C12 ayant des extrémités tangentielles à l'arc de cercle C11 et un arc de cercle C13 opposé à l'arc C11 par rapport au centre O,

— l'arc de cercle C13 ayant un centre O13 de coordonnées $X3 = L/(2\sqrt{2})$, X3, et un rayon égal à $L - X3$, et s'étendant tangentiellement entre les deux côtés adjacents du carré SQ dans le premier quadrant du repère, et

— un quatrième arc de cercle C14 issu d'un cercle symétrique de celui de l'arc de cercle C12 par rapport au centre O, ayant un centre O14 de coordonnées X2, $-X2$ et un rayon égal à $L + X2$, et s'étendant tangentiellement à partir de l'arc de cercle C13 jusqu'à la seconde diagonale D2.

Entre les boucles elliptiques E1 et E2, le filtre FI présente une transition constituée par un quart CT d'un cercle ayant comme centre le point O, et un rayon égal à $b = L + X2(1 - \sqrt{2})$, et sécant de l'axe Ox et s'étendant tangentiellement entre l'arc C14 et un premier arc de cercle C24 de la seconde boucle E2.

La seconde boucle E2 est déduite de la première boucle E1 suite à une symétrie des arcs C14, C13 et C12 par rapport à l'axe Ox. Ainsi la seconde boucle E2 peut être décomposée à partir de l'extrémité de l'arc de transition CT sur la diagonale D1 située dans le troisième quadrant du repère Ox, Oz, en

— un arc de cercle C24 identique à l'arc C14, ayant un centre O24 de coordonnées X2, X2 et un rayon égal à $L + X2$, et s'étendant jusqu'au côté inférieur de carré SQ,

— un arc de cercle C23 identique à l'arc C13, ayant un centre O23 de coordonnées $X3 = L/(2\sqrt{2})$, $-X3$ et un rayon égal à $L - X3$, et s'étendant tangentiellement entre les deux côtés adjacents du carré SQ dans le quatrième quadrant,

— un arc de cercle C22 sensiblement identique à l'arc C12, traversant sensiblement en diagonale le premier quadrant, et ayant un centre O22 de coordonnées $-X2$, $-X2$ et un rayon égal à $L + X2$,

— un arc de cercle C21 issu d'un cercle symétrique à celui de l'arc C23 par rapport au centre O, ayant un centre O21 de coordonnées $-X3$, X3 et un rayon égal à $L - X3$, et s'étendant sensiblement sur une demi-circonférence jusqu'au troisième quadrant, en passant sous l'arc de transition CT et l'arc de cercle C11, et

— un segment rectiligne ou sensiblement convexe SR2 tangent à l'arc de cercle C21, en un point d'abscisse sensiblement égale à $-L/2$, et s'étendant jusqu'à une seconde extrémité libre M2 du filtre FI située dans le quatrième quadrant, à proximité du centre O et sensiblement symétrique de la première extrémité M1 par rapport à l'axe Ox.

Il apparaît ainsi que le filtre FI comporte deux courbes fermées convexes qui sont développées sensiblement suivant deux ellipses E1 et E2 ayant des grands axes orthogonaux de longueur $2a = 3L - L/\sqrt{2}$ et des petits axes orthogonaux de longueur $2b = 2(L + X2(1 - \sqrt{2}))$ égale au diamètre de l'arc de transition CT. Les foyers F1 et F2 des deux boucles E1 et E2 sont situées respectivement sur les diagonales D1 et D2 et à une distance du centre O égale à $c = (a^2 - b^2)^{1/2}$.

Selon une autre variante de la première réalisation, le premier arc de cercle C11 de la première boucle E1 peut avoir un rayon plus grand, par exemple égal au rayon $L - X3$ des arcs de cercle C13, C23 et C21.

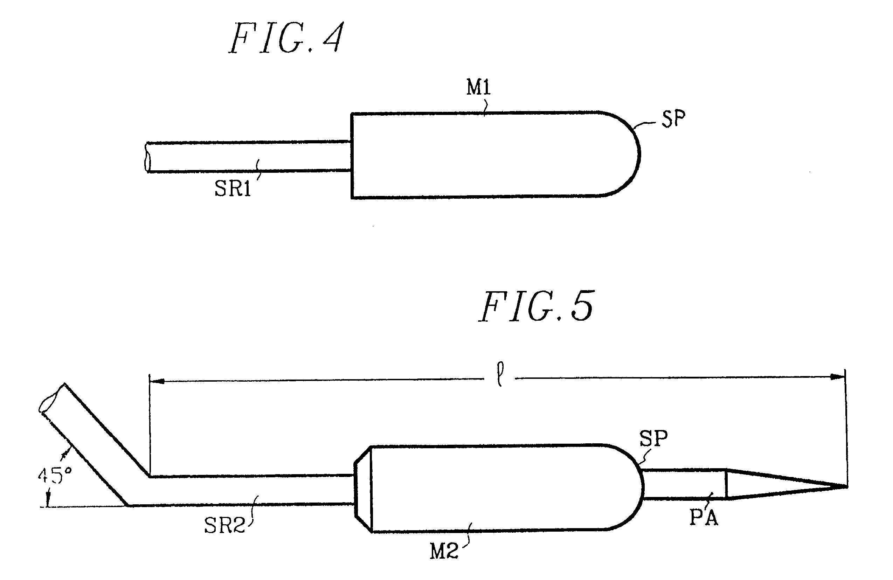

En référence aux Figs. 2 et 3, un filtre FI' selon une seconde réalisation comporte un fil ayant été soumis à une prédéformation primitive offrant deux boucles plates oblongues superposées E1' et E2'. Les boucles E1' et E2' ont des grands axes orthogonaux D1 et D2 et sont également inscrites dans un carré SQ de côté 2L et de centre O. Chacune des boucles E1' et E2' est composée sensiblement de deux demi-cercles C11' et C13', C21' et C23' ayant des centres O11' et O13', O21' et O23' équidistants de $L/\sqrt{2}$ par rapport au centre O et situés sur la diagonale respective D1, D2, et ayant des rayons égaux à $L/2$. Comme les arcs de cercle C11, C23, C13 et C21 dans la Fig. 1, les demi-cercles C11', C23', C13' et C21' sont situés dans les quatre coins du carré SQ et sont tangents chacun à deux côtés adjacents respectifs du carré. La plupart des extrémités des demi-cercles sont reliées par des segments de droite, ou des segments sensiblement convexes, parallèles aux diagonales respectives D1, D2 et ayant une longueur $L\sqrt{2}$. Chacune des boucles E1′ et E2′ a ainsi un grand axe de longueur $L(1 + \sqrt{2})$ et un petit axe de longueur L.

La première boucle E1′ est composée d'un segment SR1′ rectiligne ou sensiblement convexe entre une première extrémité libre M1′ du filtre FI′ située sensiblement sur la diagonale D1 au voisinage du centre O, dans le premier quadrant, et une extrémité du demi-cercle C11′ sensiblement sur l'axe Ox, du demi-cercle C11′, d'un segment de droite S12′ de longueur $L\sqrt{2}$ reliant les extrémités des demi-cercles C11′ et C13′ et parallèle à la diagonale D1, du demi-cercle C13′, et d'un segment de droite S14′ de longueur $L/\sqrt{2}$ parallèle à la diagonale D1 et prolongeant le demi-cercle C13′ dans le second quadrant jusqu'à la diagonale D2.

De même, la seconde boucle oblongue E2′ comporte un segment de droite S24′ de longueur $L/\sqrt{2}$ qui est parallèle à la diagonale D2 et symétrique du segment S14′ par rapport à l'axe Ox. Un quart de cercle de transition CT′ de rayon $L/2$ et de centre O relie les extrémités proches des segments S14′ et S24′.

La seconde boucle E2′ est ainsi composée du segment S24′ de longueur $L/\sqrt{2}$, du demi-cercle C23′, d'un segment de droite S22′ parallèle à la diagonale D2, ayant une longueur L et joignant des extrémités des demi-cercles C23′ et C21′ respectivement proches des axes Ox et Oz, du demi-cercle C21′, et d'un segment de droite ou sensiblement curviligne convexe SR2′ prolongeant le demi-cercle C21′ en direction du quatrième quadrant, passant sous le demi-cercle C11′ et le quart de cercle CT′, et terminé par une seconde extrémité libre M2′ du filtre FI′.

On notera que le premier filtre FI est plus facilement enroulable à sa configuration en double boucle que le second filtre F1′, grâce à un remplacement de segments rectilignes S12′, S14′, S24′ et S22′ par les grands arcs de cercle C12, C14, C24 et C22. Selon d'autres variantes, tout arc convexe de boucle peut être approximé par une ligne polygonale convexe.

Lorsqu'il est placé dans un canal physiologique, tel qu'un vaisseau sanguin ou une veine V, ayant un diamètre de section sensiblement inférieur à L, l'axe bissecteur Oz des diagonales du carré SQ colinéaires aux grands axes des boucles E1 et E2, E1′ et E2′ est confondu avec l'axe longitudinal de la veine V. En coupe longitudinale de la veine V, comme montré aux Figs. 1 et 2, la veine V est élargie sensiblement à une largeur 2L sur une longueur faible sensiblement égale à 2L n'excédant pas 40 mm environ. Ceci réduit d'autant le risque de malposition du filtre dans une veine collatérale. La longueur 2L est choisie de telle sorte qu'elle soit sensiblement inférieure à l'espace entre deux vertèbres. En pratique, la longueur 2L peut être comprise entre 25 et 36 mm. La veine V s'appuie sur le filtre en quatre points de contact P11, P23, P13 et P21, P11′, P23′, P13′ et P21′, deux-à-deux symétriques par rapport aux axes Ox et Oz, et tangentiellement aux quatre arcs de cercle C11, C23, C13 et C21, C11′, C23′, C13′ et C21′ situés aux quatre coins du carré SQ. Le filtre ainsi positionné est en équilibre permanent de tension, tout en étant relativement court dans le sens longitudinal de la veine V, ce qui évite toute migration du filtre suivant l'une ou l'autre des directions longitudinales.

Vu en section transversale, comme montré à la Fig. 3, les quatre points de contact sont également sensiblement symétriques par rapport à des axes transversaux orthogonaux Ox et Oy de la veine V. Le filtre est très plat et diminue la section de la veine d'une section circulaire avec un diamètre de l'ordre de L en une section elliptique STV de grand axe 2L et petit axe $L/4$ environ et donc ayant une excentricité très proche de l'unité. Le fil du filtre traverse six fois le petit axe Oy de la section transversale de la veine. Ces traversées conjointement au caractère plat longitudinal du filtre contribuent à arrêter tout corps étranger de taille estimée dangereuse entraîné par le flux veineux, tel que caillot sanguin ou embolie, tout en n'interrompant pas le débit sanguin, le filtre lui-même n'étant pas thrombogène.

Le filtre selon l'invention offre également une autre propriété intéressante. Le fil du filtre suit constamment un sens unique de rotation autour du centre O, de la première extrémité M1 vers la seconde extrémité M2, dans un plan longitudinal à la veine V, par exemple selon un sens de rotation contraire à celui des aiguilles d'une montre selon les réalisations montrées aux Figs. 1 et 2. Cette propriété confère au filtre, d'une part, un équilibre parfait malgré la compression exercée par la paroi veineuse, autour du centre O, ce qui évite tout déploiement du filtre dans le sens longitudinal de la veine résultant du pouvoir élastique centrifuge du fil, d'autre part, une reprise instantanée de sa configuration initiale lors du largage du filtre dans la veine. Comme on le verra dans la suite, tant que le filtre n'est pas totalement largué de l'extrémité proximale d'un cathéter, le filtre peut être déployé pour former partiellement la première boucle, puis la seconde boucle, ou être partiellement ou totalement retiré de la veine afin de le positionner convenablement.

A titre d'exemple, le fil du filtre a un diamètre de 0,3 mm, et sa longueur varie de 160 à 260 mm environ, selon la longueur 2L choisie. La prédéformation initiale du fil est obtenue selon un procédé connu consistant en une prédéformation à froid du fil selon la configuration choisie au moyen de cylindres calibreurs ayant des rayons sensiblement inférieurs à ceux des différents arcs de cercle des boucles, puis en une trempe après avoir porté le fil à une température de 500°C pendant trois heures environ. Cette trempe augmente la dureté du fil.

Comme montré à la Fig. 4, la première extrémité, dite extrémité proximale, du filtre FI, FI′ qui vient la première en contact avec la veine V lors de l'opération de largage, est conformée en une petite masselotte de révolution M1, M1', ici cylindrique et coaxiale au fil, ayant typiquement une longueur de 4 mm. Le diamètre de la masselotte M1', M1 est compris entre 0,6 et 1 mm, afin que la masselotte puisse glisser librement dans la veine V et dans un cathéter normalisé, par exemple de taille 7F de diamètre interne 1,45 mm. L'extrémité de la masselotte M1, M1' à l'opposé du fil, comporte un arrondi hémisphérique SP pour faciliter la pénétration du filtre dans la veine V sans agresser la veine et donc l'endothélium veineux, tout en offrant une surface de contact avec celui-ci relativement grande.

Comme montré à la Fig. 5, la seconde extrémité du filtre FI, FI', qui pénètre en dernier dans le cathéter et la veine, comporte également une masselotte de révolution M2, M2', ici cylindrique et coaxiale au fil et ayant une longueur égale à celle de la masselotte M1, M1', mais un diamètre plus petit, typiquement de 0,6 mm. Toutefois la seconde masselotte M2, M2' est prolongée par une pointe axiale acérée PA, conique ou biseautée, ayant typiquement un diamètre de 0,3 mm et une longueur de 3 mm. Cette pointe PA est destinée à se ficher légèrement dans la paroi de la veine V après le largage du filtre et ainsi à ancrer le filtre selon sa configuration primitive dans la veine à la position choisie. En outre, cette pointe PA avec la masselotte M2, M2' augmente la surface d'encastrement de la seconde extrémité du filtre dans un poussoir de cathéter, comme on le verra dans suite.

De préférence, comme montré aux Figs. 3 et 5, la seconde extrémité du filtre en vue transversale est inclinée d'un angle de 45° environ par rapport au segment distal SR2, SR2', et plus généralement, d'un angle de 45° environ par rapport au plan longitudinal Ox, Oz des boucles E1 et E2, E1' et E2', sur une longueur $\ell$ d'une dizaine de millimètres afin de pénétrer en biais dans la paroi veineuse et donc sur une longueur plus grande que si la pointe PA pénétrait perpendiculairement à la paroi veineuse. L'arrondi SP de la seconde masselotte M2, M2' bute contre la paroi veineuse et limite la pénétration de la pointe PA.

Les masselottes sont rapportées coaxialement aux extrémités du fil du filtre, par exemple par microsoudures, et la pointe PA peut être une extrémité usinée du fil. Au lieu d'être cylindriques, les masselottes peuvent être effilées sensiblement en tronc de cône ou en ellipsoïde.

Pour implanter le filtre, tel que le filtre FI, l'invention fait appel à un cathéter classique 3, typiquement de taille 7F, c'est-à-dire ayant des diamètres interne et externe de 1,45 mm et 2,3 mm, et une longueur de 1 m environ. Le cathéter 3 est un long tube en copolymères ayant une paroi mince offrant un faible coefficient de friction pour un coulissement aisé d'un poussoir de filtre 4 selon l'invention. Le cathéter comprend une embase cylindrique à son extrémité distale par laquelle est introduit le filtre puis est glissé le poussoir.

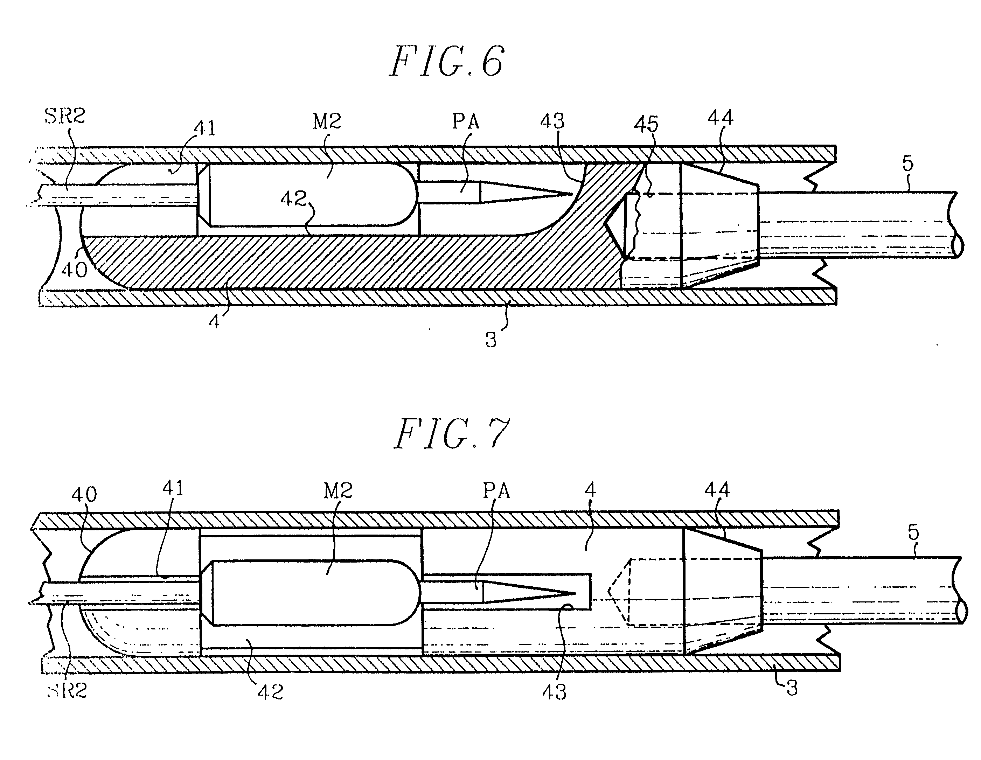

Comme montré en détail aux Figs. 6 et 7, le poussoir 4 selon l'invention destiné à recevoir la seconde extrémité M2 + PA du filtre FI est issu d'un embout cylindrique plein ayant un diamètre externe sensiblement inférieur au diamètre interne du cathéter et typiquement égal à 1,3 mm, et ayant une longueur sensiblement égale au double de la longueur de l'ensemble masselotte M2 et pointe acérée PA.

A partir d'une première extrémité hémisphérique 40 du poussoir qui pénètre en premier dans le cathéter 3, le poussoir 4 comporte une petite rainure longitudinale 41, une encoche longitudinale ouverte latéralement 42, et une seconde rainure longitudinale borgne 43. Les rainures 41 et 43 sont colinéaires suivant un plan longitudinal et axial du poussoir 4, ont une profondeur supérieure au diamètre de la masselotte M1, et typiquement égale à la somme des rayons du poussoir et du fil de filtre FI, et une largeur sensiblement supérieure au diamètre du fil de filtre. Le fond de l'encoche 42 est plat et perpendiculaire aux parois des rainures 41 et 43. La profondeur de l'encoche 42 peut être égale ou supérieure à celle des rainures. La longueur de l'encoche 42 et la longueur du fond rectiligne de la rainure 43 sont respectivement sensiblement supérieures à celles de la masselotte M2 et de la pointe PA. La longueur de la première rainure 41 est de l'ordre de 2 mm.

A une seconde extrémité tronconique 44 du poussoir 4 est prévu un trou borgne axial 45 pour recevoir à force et par collage une extrémité d'un fil tressé d'acier inoxydable 5 ayant un diamètre typiquement de 0,8 mm et nettement plus long que le cathéter 3.

L'implantation proprement dite du filtre FI succède à l'introduction du cathéter 3, d'une manière classique, par voie percutanée et anesthésie locale, par exemple à partir de la veine fémorale, humérale, céphalique ou jugulaire, au moyen d'un fil introducteur de guidage à extrémité proximale munie d'un guide cylindrique téflonné qui est poussé dans la veine jusqu'à l'endroit d'implantation choisi. Le cathéter coulisse le long du fil de guidage, puis le fil de guidage est retiré de l'intérieur du cathéter. L'extrémité proximale du cathéter est ainsi localisée devant l'endroit d'implantation choisi, par contrôle radioscopique, comme toute l'implantation du filtre FI décrit ci-après.

La première extrémité à masselotte M1 du filtre FI est introduite dans l'embase cylindrique distale externe du cathéter tubulaire 3. Puis l'extrémité M1 glisse dans le cathéter en exerçant de nombreuses poussées manuelles sur le fil du filtre qui est amené au fur et à mesure à un état sensiblement rectiligne et qui suit le parcours sinueux du cathéter. Cet acheminement du filtre FI dans le cathéter se poursuit jusqu'à ce que la seconde extrémité M2 + PA du filtre soit devant l'embase distale du cathéter.

A ce stade, si ce n'est déjà fait, la seconde masselotte M2 est logée dans l'encoche 42 du poussoir 4, et simultanément la pointe PA et l'extrémité du segment filaire SR2 du filtre FI sont logées respectivement dans les rainures 43 et 41, de sorte que l'extrémité M2 + PA ne dépasse pas radialement du poussoir 4. L'extrémité distale M2 + PA ainsi maintenue dans le poussoir 4 et momentanément solidaire de celui-ci est introduite, à l'aide d'un manchon introducteur conique rectiligne, dans le cathéter 3. Le poussoir avec l'extrémité distale du filtre glisse du manchon introducteur dans l'extrémité distale du cathéter.

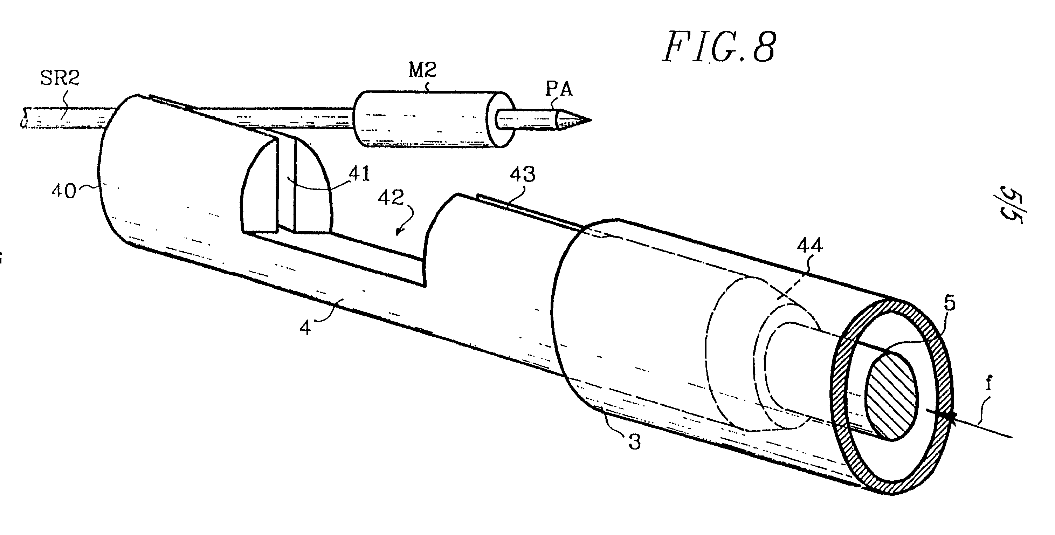

Le filtre FI continue alors à glisser dans le cathéter 3 en direction de l'extrémité proximale du cathéter dans la veine, et le poussoir 4 coulisse dans le cathéter, suivant la flèche f dans la Fig. 8, grâce à des poussées manuelles successives exercées sur le fil de poussoir 5 qui entre à son tour dans le cathéter. Lorsque l'extrémité proximale M1 du filtre débouche du cathéter 3 dans la veine V, le fil du filtre reprend progressivement sa configuration primitive en deux boucles orthogonales E1 et E2 dans la veine V.

L'emplacement du filtre peut être ajusté, par exemple en ne libérant que la longueur du filtre correspondant à la première boucle E1, et en tirant et poussant sur une faible longueur le fil de poussoir 5 qui est, à ce stade, complètement solidaire du filtre FI grâce à l'encastrement de l'extrémité M2 + PA dans le poussoir 4. Le cas échéant, le filtre peut être récupérer en retirant complètement le fil 5 et le poussoir 4 du cathéter 3, ou bien, si nécessaire, le cathéter 3 est légèrement tiré ou poussé pour positionner précisément l'extrémité proximale du cathéter devant le lieu choisi d'implantation du filtre, par exemple entre deux vertèbres déterminées, le long de la veine cave inférieure.

Finalement, le filtre FI est largué, comme montré à la Fig. 8, dès que l'extrémité à encoche 42 et rainures 41, 43 du poussoir 4 sort de l'extrémité proximale du cathéter 3, et plus précisément, dès que la rainure borgne 43 pénètre dans la veine. Toutefois, l'autre extrémité 44 du poussoir, derrière la rainure 43, demeure dans le cathéter afin de faciliter le retrait du poussoir 4 du cathéter 3, en tirant le fil de poussoir 5 suivant le sens contraire à la flèche f. Grâce au pouvoir élastique du fil de filtre, le filtre FI se déforme pour reprendre sa configuration initiale en deux boucles, progressivement au cours de sa pénétration dans la veine, et l'extrémité M2 + PA est dégagée automatiquement du poussoir pour s'arrêter contre la paroi veineuse, comme montré à la Fig. 3. La pointe PA localisée dans un plan perpendiculaire au plan des boucles du filtre pénètre sensiblement sous une incidence de 45° environ dans la paroi veineuse sans toutefois traverser celle-ci grâce à l'extrémité arrondie SP de la seconde masselotte M2 qui bute contre la paroi veineuse. Le filtre FI est ainsi définitivement implanté dans la veine V.

Après quelques jours, une endothélialisation se produit. Les portions périphériques des boucles E1, E2, au niveau des points de contact P11, P23, P13 et P21, ainsi que l'extrémité distale M2 + PA du filtre s'intègrent dans la paroi veineuse, donnant au filtre une stabilité définitive et empêchant tout risque de migration du filtre dans la veine, notamment vers le coeur et les artères pulmonaires.

Selon une autre variante, l'extrémité distale externe du cathéter 3 peut comporter un branchement en Y, dont une première branche est colinéaire au reste du cathéter pour introduire le filtre FI et le poussoir 4, et dont une autre branche latérale est inclinée par rapport à la première branche pour injecter un produit liquide lubrifiant, de préférence radio-opaque, améliorant le glissement du poussoir 4 dans le cathéter 3, et contribuant à repérer la position de l'extrémité proximale du cathéter dans la veine V.

## Revendications

1. Filtre anti-embolique (FI) à implanter dans un canal physiologique (V), qui comprend un fil flexible ayant été soumis à une prédéformation primitive en des première et seconde boucles, caractérisé en ce que lesdites boucles sont superposées sensiblement plates (E1, E2) et sensiblement symétriques respectivement par rapport à deux axes (D1, D2) qui sont sensiblement orthogonaux et concourants en un centre (O) du filtre.

2. Filtre conforme à la revendication 1, caractérisé en ce que lesdites boucles (E1, E2) sont inscrites dans un carré (SQ) ayant lesdits axes (D1, D2) pour diagonales et ayant des côtés de longueur prédéterminée 2L.

3. Filtre conforme à la revendication 2, caractérisé en ce que l'épaisseur du filtre est sensiblement égale ou inférieure à L/4.

4. Filtre conforme à la revendication 2 ou 3, caractérisé en ce que chacune des boucles (E1, E2) est tangentielle à deux côtés parallèles du carré (SQ) en deux points de contact (P11, P13 ; P21, P23) sensiblement symétriques par rapport au centre (O).

5. Filtre conforme à l'une quelconque des revendications 2 à 4, caractérisé en ce qu'en vue transversale aux boucles (E1, E2), le filtre est sensiblement circonscrit par une ellipse transversale (STV) de grand axe 2L et de petit axe L/4 environ.

6. Filtre conforme à la revendication 5, caractérisé en ce qu'il comporte quatre points de contact de boucle (P11, P23, P13, P21) avec l'ellipse transversale (STV).

7. Filtre conforme à l'une quelconque des revendications 1 à 6, caractérisé en ce que les première et seconde boucles (E1, E2) sont convexes et développées d'une première extrémité libre (M1) vers une seconde extrémité libre (M2) du filtre suivant un même sens de rotation autour du centre (O).

8. Filtre conforme à l'une quelconque des revendications 1 à 7, caractérisé en ce que lesdites boucles (E1, E2) sont elliptiques.

9. Filtre conforme à la revendication 8, caractérisé en ce que chacune des boucles elliptiques (E1, E2) a un grand axe sensiblement égal à $3L - L/\sqrt{2}$ et un petit axe sensiblement égal à $2(L + X2(1 - \sqrt{2}))$, où 2L est la longueur des côtés d'un carré (SQ) dans lequel sont inscrites lesdites boucles, et X2 est égal à $(L/4)(7 - 4\sqrt{2})^{1/2}$.

10. Filtre conforme à l'une quelconque des revendications 1 à 7, caractérisé en ce que lesdites boucles (E1', E2') sont oblongues.

11. Filtre conforme à la revendication 10, caractérisé en ce que chacune des boucles oblongues (E1', E2') a un grand axe sensiblement égal à 2L et un petit axe sensiblement égal à L, où 2L est la longueur des côtés d'un carré (SQ) dans lequel sont inscrites lesdites boucles.

12. Filtre conforme à l'une quelconque des revendications 8 ou 11, caractérisé en ce que lesdites boucles (E1, E2 ; E1', E2') sont raccordées entre elles sensiblement par un quart de cercle de fil (CT, CT') concentrique au centre (O).

13. Filtre conforme à l'une quelconque des revendications 1 à 12, caractérisé en ce que des extrémités libres (M1, M2) du filtre (FI) constituées par une première extrémité de la première boucle (E1) et une seconde extrémité de la seconde boucle (E2) sont situées à l'intérieur des boucles, de préférence au voisinage du centre (O).

14. Filtre conforme à l'une quelconque des revendications 1 à 13, caractérisé en ce que des extrémités libres des première et seconde boucles (E1, E2) comportent des première et seconde masselottes de révolution (M1, M2), respectivement.

15. Filtre conforme à la revendication 14, caractérisé en ce que les masselottes (M1, M2) ont des diamètres inférieurs à 1,45 mm.

16. Filtre conforme à la revendication 14 ou 15, caractérisé en ce que la seconde masselotte (M2) est prolongée par une pointe (PA).

17. Filtre conforme à l'une quelconque des revendications 14 à 17, caractérisé en ce que l'axe de la seconde masselotte (M2) est incliné d'environ 45° par rapport au plan (Ox, Oz) des boucles (E1, E2).

18. Poussoir destiné à glisser dans un cathéter (3) et à recevoir l'extrémité libre (M2, PA) de la seconde boucle du filtre (FI) conforme à l'une quelconque des revendications 14 à 17, caractérisé en ce qu'il est constitué par un embout cylindrique (4) comportant à partir de l'une (40) de ses extrémités, une première rainure longitudinale (41) pour recevoir une faible longueur du fil derrière la seconde masselotte (M2), et une encoche longitudinale (42) pour recevoir la seconde masselotte (M2) afin que la seconde masselotte soit encastrée complètement dans l'embout (4).

19. Poussoir conforme à la revendication 18, caractérisé en ce que l'embout comporte une seconde rainure (43) colinéaire avec la première rainure (41) et débouchant dans l'encoche (42) pour recevoir une pointe (PA) prolongeant la seconde masselotte (M2).

20. Poussoir conforme à la revendication 18 ou 19, caractérisé en ce qu'il a un diamètre sensiblement égal à 1,3 mm pour être introduit dans un cathéter (3; type 7F) ayant des diamètres interne et externe de l'ordre de 1,45 mm et 2,3 mm.

## Claims

1. Anti-embolic filter (FI) to be implanted in a physiological canal (V), which comprises a flexible wire having been subjected to a primitive predeformation into first and second loops, characterized in that said loops are substantially flat superposed (E1, E2) and substantially symmetrical respectively with regard to two axes (D1, D2) which are substantially orthogonal and convergent at a centre (O) of the filter.

2. Filter according to claim 1, characterized in that said loops (E1, E2) are inscribed in a square (SQ) having said axes (D1, D2) as diagonals and having sides of predetermined length 2L.

3. Filter according to claim 2, characterized in that the thickness of the filter is substantially equal to or less than L/4.

4. Filter according to claim 2 or 3, characterized in that each of the loops (E1, E2) is tangential to two parallel sides of the square (SQ) at two contact points (P11, P13 ; P21, P23) substantially symmetrical with regard to the centre (O).

5. Filter according to any one of claims 2 to 4, characterized in that in a transversal view of the loops (E1, E2), the filter is substantially circumscribed by a transversal ellipse (STV) of major axis 2L and minor axis L/4 approximately.

6. Filter according to claim 5, characterized in that it comprises four loop contact points (P11, P23, P13, P21) with the transversal ellipse (STV).

7. Filter according to any one of claims 1 to 6, characterized in that the first and second loops (E1, E2) are convex and developed from a first free extremity (M1) towards a second free extremity (M2) of the filter according to a same rotation direction around the centre (O).

8. Filter according to any one claims 1 to 7, characterized in that said loops (E1, E2) are elliptic.

9. Filter according to claim 8, characterized in that each of the elliptic loops (E1, E2) has a major axis substantially equal to $3L - L/\sqrt{2}$ and a minor axis substantially equal to $2(L + X2(1 - \sqrt{2}))$, whereby 2L is the length of the sides of a square (SQ) in which said loops are inscribed, and X2 is equal to $(L/4)(7 -$

$4\sqrt{2})^{1/2}$.

10. Filter according to any one of claims 1 to 5, characterized in that said loops (E1′, E2′) are oblong.

11. Filter according to claim 10, characterized in that each of the oblong loops (E1′, E2′) has a major axis substantially equal to 2L and a minor axis substantially equal to L, whereby 2L is the length of the sides of a square (SQ) in which said loops are inscribed.

12. Filter according to any one of claims 8 or 11, characterized in that said loops (E1, E2 ; E1′,E2′) are connected therebetween substantially by a quarter of a wire circle (CT, CT′) concentric at the centre (O).

13. Filter according to any one of claims 1 to 12, characterized in that free extremities (M1, M2) of the filter (FI) comprised of a first extremity of the first loop (E1) and a second extremity of the second loop (E2) are located inside the loops, preferably in the region of the centre (O).

14. Filter according to any one of claims 1 to 13, characterized in that free extremities of the first and second loops (E1, E2) comprise first and second small revolution weights (M1, M2), respectively.

15. Filter according to claim 14, characterized in that the weights (M1, M2) have diameters less than 1.45 mm.

16. Filter according to claim 14 or 15, characterized in that the second weight (M2) is prolonged by a tip (PA).

17. Filter according to any one of claims 14 to 17, characterized in that the axis of the second weight (M2) is inclined by approximately 45° with regard to the plane (Ox, Oz) of the loops (E1, E2).

18. Pusher intended to slide in a catheter (3) and to receive the free extremity (M2, PA) of the second loop of the filter (FI) according to any one of claims 14 to 17, characterized in that it is constituted by a cylindrical socket (4) comprising from the one (40) of its extremities, a first longitudinal groove (41) for receiving a short length of the wire behind the second weight (M2), and a longitudinal notch (42) for receiving the second weight (M2) whereby the second weight be completely embedded in the socket (4).

19. Pusher according to claim 18, characterized in that the socket comprises a second groove (43) colinear with the first groove (41) and emerging into the notch (42) for receiving a tip (PA) prolonging the second weight (M2).

20. A pusher according to claim 18 or 19, characterized in that it has a diameter substantially equal to 1.3 mm thereby introducing it into a catheter (3 ; type 7F) having internal and external diameters of approximately 1.45 mm and 2.3 mm.

**Patentansprüche**

1. Antiemboliefilter (FI) zur Implantation in einem physiologischen Kanal (V), welches einen flexiblen Draht aufweist, der zur Bildung einer ersten und einer zweiten Schleife eine Anfangs-Vordeformation erfahren hat, dadurch gekennzeichnet, daß die Schleifen im wesentlichen eben (E1, E2) sowie im wesentlichen symmetrisch bezüglich zweier Achsen (D1, D2) übereinandergelegt sind, die im wesentlichen orthogonal sind und in einem Zentrum (O) des Filters zusammenlaufen.

2. Filter nach Anspruch 1, dadurch gekennzeichnet, daß die Schleifen (E1, E2) in ein Rechteck (SQ) einbeschrieben sind, welches die Achsen (D1, D2) als Diagonale und Seiten vorbestimmter Länge 2L hat.

3. Filter nach Anspruch 2, dadurch gekennzeichnet, daß die Dicke des Filters im wesentlichen gleich oder weniger als L/4 beträgt.

4. Filter nach Anspruch 2 oder 3, dadurch gekennzeichnet, daß jede Schleife (E1, E2) tangential zu zwei parallelen Seiten des Rechtecks (SQ) mit zwei Berührungspunkten (P11, P13 ; P21, P23) verläuft, welche im wesentlichen symmetrisch zum Zentrum (O) liegen.

5. Filter nach einem der Ansprüche 2 bis 4, dadurch gekennzeichnet, daß in Queransicht der Schleifen (E1, E2) das Filter im wesentlichen von einer querliegenden Ellipse (STV) mit einer großen Achse von etwa 2L und einer kleinen Achse von etwa L/4 umschrieben ist.

6. Filter nach Anspruch 5, dadurch gekennzeichnet, daß vier Schleifen-Berührungspunkte (P11, P23, P13, P21) mit der querliegenden Ellipse (STV) vorhanden sind.

7. Filter nach einem der Ansprüche 1 bis 6, dadurch gekennzeichnet, daß die ersten und zweiten Schleifen (E1, E2) konvex und aus einer ersten freien Extremität (M1) nach einer zweiten freien Extremität (M2) des Filters in gleichem Drehsinn um das Zentrum (O) entwickelt sind.

8. Filter nach einem der Ansprüche 1 bis 7, dadurch gekennzeichnet, daß die Schleifen (E1, E2) elliptisch sind.

9. Filter nach Anspruch 8, dadurch gekennzeichnet, daß jede elliptische Schleife (E1, E2) eine große Achse von im wesentlichen gleich $3L - L/\sqrt{2}$ und eine kleine Achse von im wesentlichen gleich $2(L + X2(1 - \sqrt{2}))$ hat, wobei 2L eine Seitenlänge des Rechtecks (SQ) ist, in welches die Schleifen einbeschrieben sind, und wobei $X2 = (L/4)(7 - 4\sqrt{2})^{1/2}$ ist.

10. Filter nach einem der Ansprüche 1 bis 7, dadurch gekennzeichnet, daß die Schleifen (E1′, E2′) länglich sind.

11. Filter nach Anspruch 10, dadurch gekennzeichnet, daß jede längliche Schleife (E1′, E2′) eine große Achse von im wesentlichen gleich 2L und eine kleine Achse von im wesentliche L hat, wobei 2L die Seitenlänge eines Rechtecks (SQ) ist, in welches die Schleifen einbeschrieben sind.

12. Filter nach einem der Ansprüche 8 bis 11, dadurch gekennzeichnet, daß die Schleifen (E1, E2 ; E1′, E2′) untereinander im wesentlichen durch einen zum Zentrum (O) konzentrischen Viertelkreis des Drahtes (CT, CT′) verbunden sind.

13. Filter nach einem der Ansprüche 1 bis 12, dadurch gekennzeichnet, daß die freien Extremitäten (M1, M2) des Filters (FI), welche durch eine erste Extremität der ersten Schleife (E1) und eine zweite Extremität der zweiten Schleife (E2) gebildet sind, im Innern der Schleifen, bevorzugt in der Nähe des Zentrums (O) angeordnet sind.

14. Filter nach einem der Ansprüche 1 bis 13, dadurch gekennzeichnet, daß die freien Extremitäten der ersten und zweiten Schleifen (E1, E2) jeweils erste und zweite Drehköpfe (M1, M2) aufweisen.

15. Filter nach Anspruch 14, dadurch gekennzeichnet, daß die Drehköpfe (M1, M2) Durchmesser haben, die kleiner als 1,45 mm sind.

16. Filter nach Anspruch 14 oder 15, dadurch gekennzeichnet, daß der zweite Drehkopf (M2) durch eine Spitze (PA) verlängert ist.

17. Filter nach einem der Ansprüche 14 bis 17, dadurch gekennzeichnet, daß die Achse des zweiten Drehkopfes (M2) ungefähr um 45° gegen die Ebene (Ox, Oz) der Schleifen (E1, E2) geneigt ist.

18. Schieber, der dazu bestimmt ist, in einen Katheder (3) zu gleiten und die freie Extremität (M2, PA) der zweiten Schleife des Filters (FI) nach einem der Ansprüche 14 bis 17 aufzunehmen, gekennzeichnet durch einen zylindrischen Ansatz (4), der, ausgehend von einem (40) seiner Enden, eine erste Längsschiene (41) zur Aufnahme einer kleinen Länge des Drahtes hinter dem zweiten Drehkopf (M2) und eine Längnut (42) zur Aufnahme des zweiten Drehkopfes (M2) aufweist, um den zweiten Drehkopf vollständig in dem Ansatz (4) zu versenken.

19. Schieber nach Anspruch 18, dadurch gekennzeichnet, daß der Ansatz (4) eine zweite Schiene (43), die mit der ersten Schiene (41) kolinear ist und in die Nut (42) mündet, aufweist, um eine den zweiten Drehkopf (M2) verlängernde Spitze (PA) aufzunehmen.

20. Schieber nach Anspruch 18 oder 19, dadurch gekennzeichnet, daß er einen Durchmesser von im wesentlichen gleich 1,3 mm besitzt, um in einen Katheder (3 ; Typ 7F) einführbar zu sein, welcher Innen- und Außendurchmesser in der Größenordnung von 1,45 mm und 2,3 mm aufweist.

# FIG.1

## *FIG.2*

## *FIG.3*

## FIG.4

M1

SP

SR1

## FIG.5

$\ell$

SP

45°

SR2

M2

PA

EP 0 323 333 B1

FIG.6

FIG.7

5/5

FIG.8